# FASCICULE DE BREVET EUROPEEN

(11) **EP 3 285 888 B1**
(45) Date de publication et mention de la délivrance du brevet: **21.08.2019**
(21) Numéro de dépôt: 16720461.9
(22) Date de dépôt: 15.04.2016
(51) Int. Cl.: A61Q 17/00, A61K 8/97, A61K 8/96

(54) **UTILISATION COSMETIQUE D'UN EXTRAIT DE DIOSPYROS MESPILIFORMIS SEUL OU EN ASSOCIATION AVEC D'AUTRES ACTIFS COMME AGENT ANTI-POLLUTION**
KOSMETISCHEVERWENDUNG EINES EXTRAKTS AUS DIOSPYROS MESPILIFORMIS ALEINE ODER KOMBINIERT MIT ANDEREN WIRKSTOFFEN ZUM SCHUTZ GEGEN DIE UMWELTVERSCHMUTZUNG
COSMETIC USE OF A DIOSPYROS MESPILIFORMIS ALONE OR IN COMBINATION WITH OTHER ACTIVE AGENTS AS ANTI-POLLUTION AGENT

(30) Priorité: 20.04.2015 FR 1553518
(43) Date de publication de la demande: 28.02.2018
(73) Titulaire: Laboratoires Clarins, 75823 Paris Cedex 17 (FR)
(72) Inventeur: COURTIN, Olivier, 92200 Neuilly Sur Seine (FR); FITOUSSI, Richard, 78600 Maisons Laffitte (FR); WEBER, Sandrine, 95830 Cormeilles En Vexin (FR)
(74) Mandataire: Gevers & Orès
(86) Numéro de dépôt international: PCT/FR2016/050895
(87) Numéro de publication internationale: WO 2016/170256

(56) Documents cités:
- US-A1- 2009 035 235
- US-A1- 2013 216 635
- DATABASE GNPD [Online] MINTEL; mars 2015 (2015-03), "Anti-Pollution Day Screen Multi-Protection SPF 50", XP002753047, Database accession no. 3066485
- DATABASE GNPD [Online] MINTEL; mai 2012 (2012-05), "Intense Hydration Youth Preserve Light Day Cream SPF 15", XP002753048, Database accession no. 1798499
- DATABASE GNPD [Online] MINTEL; juin 2010 (2010-06), "Anti-Dandruff Jackberry Hairwash", XP002753049, Database accession no. 1351170
- MABONA UNATHI ET AL: "Antimicrobial activity of southern African medicinal plants with dermatological relevance: From an ethnopharmacological screening approach, to combination studies and the isolation of a bioactive compound", JOURNAL OF ETHNOPHARMACOLOGY, ELSEVIER IRELAND LTD, IE, vol. 148, no. 1, 30 mars 2013 (2013-03-30) , pages 45-55, XP028554035, ISSN: 0378-8741, DOI: 10.1016/J.JEP.2013.03.056
- DANGOGGO ET AL: "Phytochemical Analysis and Antibacterial Screening of Leaves of Diospyros mespiliformis and Ziziphus Spina-Christi", J CHEM ENGINEERING, vol. 1, no. 1, janvier 2012 (2012-01), pages 31-37, XP002753050,
- MOHAMED ET AL: "Bioactive Natural Products from Two Sudanese medicinal Plants Diospyros mespiliformis and Croton zambesicus", REC. NAT. PROD., vol. 3, no. 4, 2009, pages 198-203, XP002753051,

## Description

La présente invention se rapporte à l'utilisation cosmétique d'un extrait de *Diospyros mespiliformis* seul ou en association avec un extrait de Sanicle d'Europe et/un extrait de *Furcellaria lumbricalis* et/ou un extrait de *Lampsana communis* pour lutter contre et/ou prévenir les effets de la pollution sur la peau ; l'invention se rapporte également à un procédé cosmétique de prévention ou de traitement cosmétique des effets cutanés de la pollution consistant à appliquer sur la peau un extrait de *Diospyros mespiliformis* seul ou en association avec d'autres actifs.

L'air pur est un mélange de 78% d'azote, 21% d'oxygène, 0.09% d'argon. 0.1% de vapeur d'eau et d'autres gaz plus ou moins rares dont le gaz carbonique, hydrogène, l'ozone.

La pollution atmosphérique se définit par la modification de l'air pur par modification de ses composants ou par adjonction d'éléments nocifs. Très schématiquement, il est possible de considérer que les polluants actuellement répertoriés sont essentiellement produits par l'industrie, le chauffage et la circulation automobile. Il est habituel de classer les polluants en sept familles chimiques :
- les agents oxydants tels que l'ozone ou les oxydes d'azote sont irritants et générateurs de radicaux libres ;
- les poussières sont des particules en suspension fixant parfois des hydrocarbures polycycliques ; leur rôle nocif est modifié par la température et le degré d'humidité de l'air, les particules acides sont irritantes ; on observe une baisse de l'hydratation et de l'oxygénation des tissus.
- les produits chimiques organiques sont cancérigènes, ils proviennent des déchets de l'industrie et de la combustion des automobiles ;
- le monoxyde de carbone entraîne une hypoxie tissulaire, il provient à 80% des automobiles ;
- les hydrocarbures et les solvants représentent 50% de la pollution automobile, ils sont toxiques, irritants, cancérigènes et mutagènes ; en présence de lumière, ils réagissent avec les oxydes d'azote et produisent de l'ozone ;
- le dioxyde de soufre est l'un des produits de la combustion du mazout et du charbon, il provoque une altération du film hydro-lipidique de la peau et des irritations cutanées ;
- les métaux tels que le plomb, le zinc, l'aluminium, le mercure,... peuvent interférer avec le métabolisme cellulaire, par atteinte des réactions enzymatiques. Ils participent aux dommages oxydatifs avec lésions de l'ADN et des lipides cellulaires.

L'air intérieur des habitations, en continuité avec l'air extérieur, comprend également ces propres sources de polluants pouvant être d'origine biologique (sources d'humidité et de moisissures, endotoxines, allergènes, etc.), physique (radon, particules, fibres, etc.) et chimique (fumée de tabac, aldéhydes, composés organiques volatils - COV, métaux, etc.).

En 1969, Harry DanielI reconnaît que les fumeurs semblent plus vieux que les non-fumeurs. Par la suite, il développe un système de cotation des rides, ce qui lui a permis d'analyser l'association entre le tabagisme et la peau ridée plus objectivement avec ce système, il a pu valider sa première impression. Par la suite, son système a été utilisé dans diverses études épidémiologiques (Daniell HW. Smoker's wrinkles. A study in the epidemiology of "crow's feet". Ann Intern Med 1971; 75:873-80).

Ces études épidémiologiques ont prouvé que le tabagisme chronique était un important facteur environnemental impliqué dans le vieillissement cutané précoce (Monta A. Tabacco smoke causes prématuré skin aging. J Dermatol Sci 2007; 48:169-75). Les mécanismes physiopathologiques évoqués sont nombreux. La déshydratation du *stratum corneum* par la fumée de tabac expliquerait en partie la diminution de l'épaisseur de la couche cornée (Freiman A et al. Cutaneous effects of smoking. J Cutan Med Surg 2004; 8:415-23). En libérant des thiocyanates, en augmentant le taux de carboxyhémoglobine et en favorisant la sécrétion de vasopressine, le tabagisme chronique est à l'origine d'une ischémie chronique relative du derme, avec diminution de la pression partielle transcutanée en O₂. De ce fait, le métabolisme dermique est modifié : les fibres élastiques dystrophiques et protéoglycanes s'accumulent, les protéines de la matrice extracellulaire sont davantage dégradées par les métalloprotéases et la synthèse du collagène est diminuée.

L'association entre le tabagisme et le vieillissement de la peau semble être médiée à travers une expression plus élevée d'ARNm de la MMP-1 et de la MMP-3 ainsi que d'une diminution des collagènes I et III (Yin L et al. Skin aging induced by ultraviolet exposure and tobacco smoking: evidence from epidemiological and molecular studies. Photodermatol Photoimmunol Photomed 2001; 17:178-83: Yin L et al. Altérations of extracellular matrix induced by tobacco smoke extract. Arch Dermatol Res 2000; 292:188-94).

Le tabagisme semble également être associé à une augmentation de l'élastose et des télangiectasies (Kennedy C et al. Effect of smoking and sun on the aging skin J Invest Dermatol 2003; 120:548-54) indiquant d'autres voies moléculaires en plus de l'induction de l'expression de MMP-1. Récemment, il a été suggéré que la voie AhR peut jouer un rôle dans le processus de vieillissement prématuré de la peau induit par le tabac (Morita A et al. Molecular basis of tobacco smoke-induced premature skin aging. J Investig Dermatol Symp Proc 2009; 14:53-5).

La peau, organe dont la fonction première est d'être une barrière externe, est en contact direct avec les différents polluants de l'air et par conséquent le contact de la peau avec la pollution de l'air induit notamment à un vieillissement de la peau.

Certaines études ont étudié l'effet de l'ozone sur la peau ou sur les cellules cutanés. L'ozone est un polluant qui peut se former au niveau du sol à la suite d'une interaction entre le rayonnement solaire et les émissions de gaz de pot d'échappement. McCarthy *et al.* ont exposé à des concentrations d'ozone mesurées dans les villes des kératinocytes épidermiques humains normaux (NHEK) et analysé ses effets. Les niveaux de peroxyde d'hydrogène et d'IL-1α augmentent tandis que les niveaux d'ATP diminuent. Enfin, McCarthy *et al.* ont constaté que l'ozone augmente des dommages de l'ADN (McCarthy JT et al. Effects of ozone in normal human epidermal keratinocytes. Exp Dermatol. 2013 May;22(5):360-1). D'autres études ont étudié l'effet de l'ozone sur le tissu cutané murin qui montre que l'ozone puissant agent oxydant, est susceptible d'affecter l'intégrité de la peau (Thiele JJ et al. Ozone-exposure depletes vitamin E and induces lipid peroxidation in murine stratum corneum. J Invest Dermatol 1997; 108:753-7). Egalement, une étude a montré que l'ozone était capable d'induire l'expression de MMP-9 dans la peau murine indiquant un rôle dans le remodelage matriciel (Valacchi G et al. Induction of stress proteins and MMP-9 by 0.8 ppm of ozone in murine skin. Biochem Biophys Res Commun 2003; 305:741-6).

Une étude épidémiologique récente a découvert un lien direct entre l'exposition aux particules en suspension (PM) et l'apparition de signes de vieillissement de la peau comme les taches pigmentaires mais aussi les rides (Vierkötter A et al. Airborne particle exposure and extrinsic skin aging. J Invest Dermatol 2010; 130:2719-26). Un mécanisme important par lequel les particules ambiantes exercent ses effets néfastes est la génération de ROS (Donaldson K et al. Combustion-derived nanoparticles: a review of their toxicology following inhalation exposure. Part Fibre Toxicol 2005; 2:10).

En outre, les particules peuvent servir de supports pour des produits chimiques organiques et des métaux qui sont capables de se localiser dans les mitochondries pour y générer des ROS conduisant à un vieillissement de la peau par des lésions mitochondriales (Li N, Sioutas C et al. Ultrafine particulate pollutants induce oxidative stress and mitochondrial damage. Environ Health Perspect 2003; 111:455-60). Une autre voie induisant le vieillissement de la peau pourrait être celle des hydrocarbures aromatiques polycycliques (HAP). Adsorbées à la surface des particules en suspension dans l'air des zones urbaines, les HAP sont en mesure d'activer le métabolisme des xénobiotiques via AhR (Menichini E. Urban air pollution by polycyclic aromalic hydrocarbons: levels and sources of variability. Sci Total Environ 1992; 116:109-35).

D'autres travaux menés par Ushio *et al.* (1999) ont testé l'action de particules diesel sur des kératinocytes en culture. De fortes concentrations en particules diesel sont responsables d'altérations de la croissance de ces cellules et de leur migration.

Les particules auraient donc une action directe sur l'intégrité cutanée. De plus, cette exposition provoque une augmentation de la sécrétion en IL-8. Or, cette cytokine est incriminée dans les réactions cutanées inflammatoires. Cette étude semble suggérer que les particules diesel pourraient jouer un rôle dans l'initiation et/ou la pathogenèse de troubles cutanés inflammatoires tels que l'eczéma, la dermatite allergique ou le psoriasis (Ushio, H., K. Nohara, et al. (1999). "Effect of environmental pollutants on the production of pro- inflammatory cytokines by normal human dermal keratinocytes." Toxicol Lett 105(1): 17-24).

Il apparait donc que les polluants atmosphériques extérieurs et intérieurs sont la cause de nombreuses manifestations cutanées inesthétiques : notamment une altération de l'épaisseur, de l'élasticité, de la fermeté ou encore de l'éclat de la peau, une apparition de rides et d'un teint terne, voire de taches pigmentaires. Même si ces manifestations cutanées restent superficielles et ne sont pas pathologiques, la plupart des individus sont plus ou moins exposés aux polluants et il existe un fort besoin de mettre au point de nouvelles formulations cosmétiques qui permettraient de prévenir ou de freiner leur apparition.

On rappelle que la peau est un organe très étendu recouvrant toute la surface du corps et totalise chez l'homme adulte une surface d'environ 1,6 mètre carré. Elle a pour fonction de protéger les tissus profonds du milieu extérieur et a une activité notamment au niveau de l'immunité, de la régulation de la température et de la déperdition de fluides. La peau est constituée de trois parties principales. Une partie mince superficielle nommée épiderme, une partie interne et épaisse nommée derme, et une partie profonde et graisseuse nommée hypoderme. L'épiderme se caractérise essentiellement par son organisation en strate correspondant à un état de différenciation croissant des kératinocytes, cellules constituant environ 90% de l'épiderme. Ces kératinocytes migrent des couches profondes de l'épiderme vers la surface externe de l'épiderme. Durant cette migration, les kératinocytes subissent de nombreuses modifications biochimiques et structurales dont la plus importante est la kératinisation, processus par lequel les cellules synthétisent la kératine. La couche cornée, la couche la plus superficielle de l'épiderme est composée principalement de cellules mortes appelées cornéocytes est en particulier très résistante aux agressions externes. L'épiderme peut être défini comme un épithélium de revêtement qui constitue la structure externe de la peau. Il assure la protection de la peau, notamment par la production de kératine, protéine filamenteuse et résistante. La kératine est en partie responsable de l'imperméabilité de la peau.

Le derme est séparé de l'épiderme par la « jonction dermoépidermique ». Il se poursuit en profondeur sans limite franche par l'hypoderme. Le rôle du derme est notamment de maintenir la souplesse et la résistance de la peau. Il assure également d'autres fonctions essentielles. Sa vascularisation importante lui permet de réaliser des échanges nutritifs avec l'épiderme. Elle lui permet aussi d'être riche en cellules immunitaires de type macrophages et cellules dendritiques, acteurs essentiels de la défense immunitaire de la peau. De plus, c'est un tissu innervé possédant des récepteurs sensitifs responsables du sens du toucher. Enfin, il intervient dans les phénomènes de cicatrisation, et de régulation de la prolifération et de la différenciation de kératinocytes en synthétisant des cytokines et des facteurs de croissance solubles à destination de l'épiderme. Le derme est un tissu conjonctif de soutien essentiellement constitué de fibroblastes et d'un réseau microfibrillaire de collagène, d'élastine et de protéoglycanes formant la matrice extracellulaire. La matrice extracellulaire est le substrat d'adhésion des fibroblastes et le support mécanique du tissu.

Dans le cadre de recherches visant à identifier des actifs destinés à protéger et/ou à réparer les effets cutanés de la pollution atmosphérique extérieure et/ou intérieure, la Demanderesse a découvert que les extraits de *Diospyros mespiliformis* présentaient une activité marquée contre les effets cutanés du formaldéhyde, de la fumée de cigarette et des particules fines (voir les essais décrits dans l'exemple 1).

La présente invention se rapporte ainsi à l'utilisation cosmétique d'un extrait de *Diospyros mespiliformis* ou à une composition cosmétique le comprenant pour prévenir et/ou réparer les effets cutanés de la pollution : l'utilisation selon l'invention est particulièrement adaptée à une application à des peaux saines.

Dans le cadre de la présente invention, on désigne par peau saine une peau qui ne présente pas de pathologie cutanée et qui en outre est en bon état (au sens de l'article 2.1.a) du REGLEMENT (CE) N° 1223/2009 DU PARLEMENT EUROPÉEN ET DU CONSEIL du 30 novembre 2009 relatif aux produits cosmétiques).

*Diospyros mespiliformis*, aussi appelé Ebénier d'Afrique, est une plante de la famille des Ebenaceae, il s'agit d'un arbre originaire d'Afrique à feuilles persistantes pouvant atteindre 40 cm de haut avec une couronne très branchue et un feuillage dense. Les feuilles sont alternes, simples et entières et les fleurs blanches à jaune verdâtre. Son fruit est traditionnellement consommé en boisson ou en bouillie mélangé avec de la farine de maïs.

Cette plante est utilisée pour la préparation de remèdes traditionnels notamment en raison de ses propriétés antibiotiques ; elle est également utilisée pour améliorer la fertilité, traiter le paludisme, la syphilis, la lèpre, les mycoses ou encore comme vermifuge. Les décoctions de feuilles sont utilisées pour soigner la fièvre, les otites et les plaies.

A ce jour, aucune activité de cette plante pour prévenir ou réparer les effets cutanés de la pollution n'a été décrite.

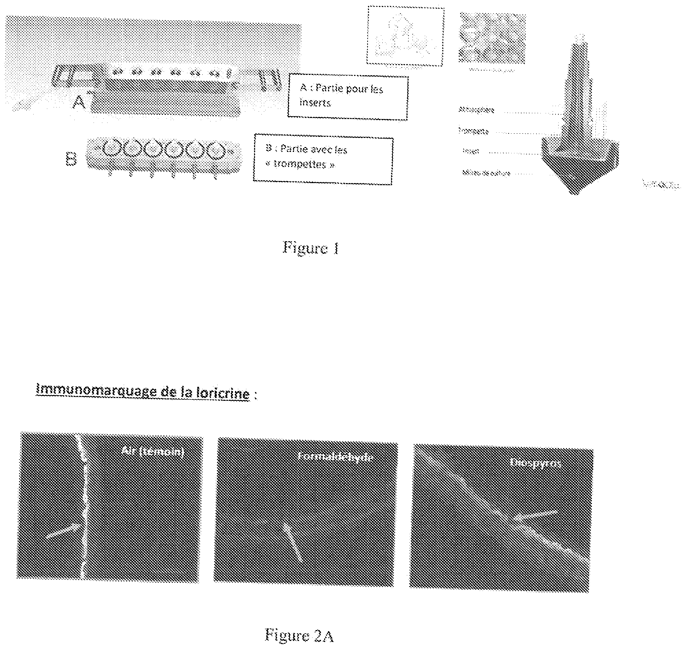

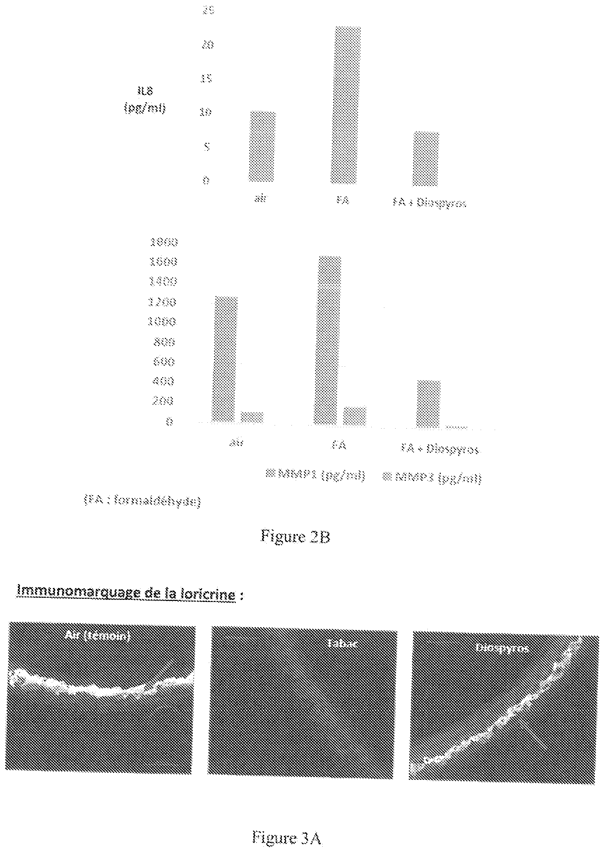

Les essais conduits, sur des modèles d'épiderme sain, traités ou non par un extrait de *Diospyros mespiliformis* et exposés à divers polluants, par la Demanderesse ont permis de mettre en évidence les activités suivantes de l'extrait de *Diospyros mespiliformis* :
- Cet extrait prévient et corrige les effets cutanés délétères du formaldéhyde en renforçant la barrière cutanée par l'induction de l'expression des marqueurs constitutifs de l'enveloppe cornée (hornerine HRNR, Small Proline Rich Protein SPRR2A, CE2B, LCE3D) ; cet effet est également confirmé par l'identification, par immunohistochimie, de l'induction de l'expression de la loricrine, protéine majoritaire de l'enveloppe cornée. En outre, cet extrait induit également l'augmentation de l'expression de SIRT1, protéine impliquée dans l'homéostasie des processus de prolifération et de différenciation des kératinocytes. Enfin, l'extrait de *Diospyros mespiliformis* protège l'épiderme soumis au formaldéhyde en réduisant les manifestations inflammatoires ; en effet, les résultats obtenus montrent que les marqueurs de l'inflammation, de l'apoptose et de la dégradation matricielle sont sous-exprimés ; le dosage de la cytokine IL-8 et des protéases matricielles MMP1, MMP9 confirment les résultats de l'étude transcriptomique.
- L'extrait de *Diospyros mespiliformis* protège l'épiderme soumis à la fumée de cigarette en ne sollicitant plus les acteurs de l'inflammation. Les résultats des essais présentés dans l'exemple 1 montrent que les marqueurs de l'inflammation, de l'apoptose et de la dégradation matricielle sont sous-exprimés en présence de l'extrait ; le dosage des cytokines IL1-α, IL-8 et de MMP1 confirment les résultats de l'étude transcriptomique. En outre, l'extrait de *Diospyros mespiliformis* permet d'assurer le maintien d'une bonne hydratation (FLG2, CD44). Les résultats obtenus montrent encore que l'utilisation de cet extrait sur l'épiderme permet de préserver et de restaurer la cohésion cellulaire et la communication cellulaire assurées notamment par la claudine 1 (CLDN1) et la desmocolline 1 (DSC1) ainsi que la jonction dermo-épidermique des effets de la fumée de cigarette (maintien ou restauration de l'expression des gènes des collagènes IV et VII). Ces essais montrent notamment que l'extrait de *Diospyros mespiliformis* contribue au maintien de l'homéostasie de la peau lorsqu'elle est exposée aux polluants.
- Enfin, l'extrait de *Diospyros mespiliformis* prévient et répare l'altération de la fonction barrière par les particules atmosphériques ; ceci résulte de la forte augmentation de l'expression de la loricrine qui joue un rôle clé dans la fonction barrière cutanée. Les essais réalisés par comparaison d'un épiderme sain altéré par la présence de particules et d'un épiderme traité avec un extrait de *Diospyros mespiliformis* avant d'être exposé à des particules, montrent que ce dernier conserve une bonne cohésion cellulaire et une bonne communication cellulaire assurées notamment par la claudine 1 (CLDN1) et la desmocolline 1 (DSC1) et voit sa jonction dermo-épidermique protégée des effets des particules (augmentation de l'expression des gènes des composants majoritaires de la jonction dermo-épidermique, collagénes IV, VII et nidogène I).

L'extrait de *Diospyros mespiliformis* utilisable selon l'invention peut provenir de toute partie de la plante ; de préférence, il s'agit des parties aériennes de la plante et encore préférentiellement des feuilles. L'extraction de l'extrait à partir de la plante ou des parties choisies peut être réalisée par broyage et extraction par solvants aqueux et/ou organiques, de préférence aqueux.

Selon un mode de réalisation particulier de l'invention, l'extrait de *Diospyros mespiliformis* utilisé selon l'invention est un extrait aqueux de feuilles stabilisé par de la maltodextrine obtenu par les étapes suivantes :
- broyage de feuilles de *Diospyros mespiliformis* ;
- extraction à l'eau ;
- séparation ;
- purification ;
- ajout de maltodextrine ;
- filtration ;
- séchage par atomisation.

L'extrait ainsi obtenu est une poudre de couleur jaune-orange à beige avec une odeur caractéristique; préparé en solution aqueuse à 1%, son pH est compris entre 5,0 et 7,0.

De préférence, l'extrait de *Diospyros mespiliformis* est formulé dans une composition cosmétique dont l'homme du métier saura adapter la composition aux propriétés physico-chimiques de l'extrait.

De préférence ladite composition cosmétique comprend de 0,01 à 10%, de préférence de 0.01 à 2%, d'extrait de *Diospyros mespiliformis* en poids de matière sèche par rapport au poids total de la composition.

De façon classique, cette composition pourra encore comprendre un ou plusieurs agents de formulation ou additifs d'usage connu et utilisés dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc. Grâce à ces connaissances en matière de cosmétique, l'homme du métier saura quels agents de formulation ajouter aux compositions de l'invention et en quelles quantités en fonction des propriétés recherchées.

Cette composition peut se présenter sous toute forme connue de l'homme du métier dans le domaine de la cosmétologie et de la dermatologie sans autre restriction galénique que l'application sur le visage et sur le corps. De façon avantageuse, elle se présente sous la forme d'un gel, d'une lotion, d'une crème, d'une émulsion, d'un lait, d'un spray, etc.

La présente invention se rapporte ainsi à l'utilisation cosmétique d'un extrait de *Diospyros mespiliformis* ou d'une composition le comprenant pour protéger la peau contre les méfaits cutanés, c'est-à-dire les manifestations indésirables visibles sur la peau, induits par la pollution atmosphérique et/ou réparer ces manifestations oudèsordres cutanés induits par la pollution atmosphérique; l'utilisation selon l'invention est avantageuse, que la pollution atmosphérique résulte de la présence de polluants intérieurs ou extérieurs, il peut s'agir en particulier d'une pollution résultant de la présence dans l'air environnant d'agents oxydants tels que l'ozone ou les oxydes d'azote, de monoxyde de carbone, d'hydrocarbures et solvants, de dioxyde de soufre, de métaux, de formaldéhyde, de fumée de cigarette, de particules ou fines poussières en suspension, ou encore d'ondes électromagnétiques.

Plus particulièrement, l'utilisation d'un extrait de *Diospyros mespiliformis* ou d'une composition le comprenant permet de prévenir l'apparition des manifestations cutanées visibles sur la peau, signes de sa dégradation et/ou de réparer les désordres cutanés induits par la pollution par les activités suivantes :
- augmentation de la cohésion cutanée et du *stratum corneum* et amélioration de la fonction barrière de la peau ;
- restauration de l'homéostasie des processus de prolifération et de différenciation des kératinocytes ;
- restauration de la matrice dermique ;
- restauration de l'hydratation cutanée ;
- amélioration de la cohésion des cellules cutanées ;
- amélioration de la communication cellulaire ;
- restauration de la jonction dermo-épidermique.

Sur la base de ces propriétés, l'utilisation de l'extrait de *Diospyros mespiliformis* ou d'une composition le comprenant est particulièrement utile pour prévenir et/ou limiter et/ou corriger le vieillissement de la peau induit par la pollution et les différentes manifestations cutanées qui lui sont associées, en particulier, prévenir et/ou corriger les rides et ridules et/ou conserver l'éclat et/ou augmenter l'hydratation et/ou réguler la desquamation et/ou maintenir ou stimuler la souplesse de la peau soumise à la pollution.

Afin d'élargir le spectre d'action de l'extrait de *Diospyros mespiliformis* ou de la composition le comprenant, il peut être avantageux de lui associer d'autres actifs cosmétiques renforçant ses effets cosmétiques et/ou présentant un ou plusieurs autres effets cosmétiques complémentaires.

Il est ainsi avantageux d'associer l'extrait de *Diospyros mespiliformis*, éventuellement dans une composition cosmétique, avec un ou plusieurs autres agents cosmétiques antioxydants et/ou anti-pollution.

De préférence, ces agents cosmétiques sont choisis parmi :
- un extrait de Sanicle d'Europe qui présente des propriétés anti-oxydantes (cf. exemple 2) et qui possède en outre la propriété de renforcer le *stratum corneum* et ainsi la fonction barrière de la peau (FR 3 007 980) ; un tel extrait est présent dans le produit *Anti-pollution Day Screen Multi-Protection SPF50* de la société CLARINS. Cet extrait permet ainsi de renforcer à la fois l'action préventive contre la pollution mais aussi la réparation de la peau par l'extrait de *Diospyros mespiliformis.*

Le Sanicle d'Europe (*Sanicula europaea*) est une plante herbacée de la famille des Apiacées (Ombellifères) poussant généralement dans les sous-bois.

C'est une plante vivace, glabre de taille moyenne (15-50 cm) qui se reconnait facilement à l'aspect globuleux de ses ombelles (3 à 5 rayons inégaux).

Elle est connue pour être riche en saponines, tanins et mucilages ; elle est utilisée en médecine traditionnelle pour ses propriétés cicatrisantes, astringentes et vulnéraires.

L'extrait de Sanicle est préférentiellement un extrait hydrosoluble des parties aériennes (feuilles, fleurs et tiges). De façon avantageuse, cet extrait est un extrait hydro alcoolique des parties aériennes et, de préférence, il est stabilisé par du glycérol.

De préférence il s'agit d'un extrait hydro alcoolique obtenu selon un procédé comprenant au moins les étapes suivantes :
- séchage des parties aériennes ;
- broyage ;
- extraction éthanol/eau (80:20) ;
- filtration ;
- concentration sous vide par évaporation des solvants d'extraction ;
- ajustement de la concentration (ajout de glycérol).

L'extrait de *Sanicula europaea* est un liquide légèrement visqueux jaune-marron et d'odeur caractéristique. Il présente les caractéristiques analytiques suivantes : pH = 3.5 - 5.0 ; densité relative : 1.050-1.200 ; indice de réfraction : 1.370 - 1.400 ; rapport plante sèche/extrait = 1/10.

De préférence la composition cosmétique utilisée selon l'invention comprend de 0,01 à 10 % d'extrait de *Sanicula europaea* en poids de matière sèche par rapport au poids total de la composition.
- un extrait de *Furcellaria lumbricalis* qui est une algue rouge de la Mer Baltique. De préférence, l'extrait comprend de l'oligofurcellarane enrichi en sels marins. L'oligofurcellarane est obtenu par dépolymérisation d'un furcellarane sulfaté (galactose et anhydrogalactose) extrait de l'algue ; à l'issue de la dépolymérisation, l'oligofurcellarane est réhydraté avec une solution enrichie en sels marins, un tel type d'extrait est vendu par la société Codif sous la dénomination Hydranov P. Un extrait de *Furcellaria lumbricalis* est utilisé pour adoucir la peau et hydrater les peaux sèches dans le produit *Intense Hydration Youth Preserve Light Day Cream SPF15* de la société ORIFLAME.

Cet extrait de *Furcellaria lumbricalis* a des propriétés hydratantes de la peau et il permet de prévenir ou corriger la déshydration cutanée due à la pollution (voir l'exemple 3).

De préférence ladite composition cosmétique comprend de 0,01 à 10%, de préférence de 0,1 à 2%, d'extrait de *Furcellaria lumbricalis* en poids de matière sèche par rapport au poids total de la composition.
- un extrait de *Lampsana communis* ; le *Lampsana communis*, de la famille des Astéracées, est une plante annuelle très répandue, poussant au bord des routes et autres sites soumis à la pollution atmosphérique. C'est une plante non exigeante et très résistante. Le document US2009/0035235 décrit sont utilisation en association avec un extraits de Camellia sinensis pour la formulation d'un complexe anti-pollution ayant des propriétés anti-oxydantes et un effet sur le métabolisme énergétique.

L'extrait de *Lampsana communis* utilisé dans la composition selon l'invention est obtenu par extraction hydro-glycolique de la partie aérienne de la plante. On obtient ainsi un liquide limpide de couleur brune à odeur caractéristique présentant les caractéristiques analytiques suivantes :
- teneur en eau : 18 à 26%
- pH : 6 à 8
- densité: 1.055 à 1.075
- indice de réfraction : 1.420 à 1.440

Lorsque cet extrait est utilisé, il est ajouté à une concentration de l'ordre de 0,01 à 5% en poids, de préférence 0,01 à 0,5% en poids et tout préférentiellement de 0,01 à 0,05% en poids.

La présente invention se rapporte encore à l'utilisation d'une des associations suivantes ou à une composition la comprenant :
- un extrait de *Diospyros mespiliformis* et un extrait de Sanicle d'Europe ;
- un extrait de *Diospyros mespiliformis* et un extrait de *Furcellaria lumbricalis* ;
- un extrait de *Diospyros mespiliformis* et un extrait de *Lampsana communis* ;
- un extrait de *Diospyros mespiliformis*, un extrait de Sanicle d'Europe et un extrait de *Furcellaria lumbricalis* ;
- un extrait de *Diospyros mespiliformis*, un extrait de Sanicle d'Europe et un extrait de *Lampsana communis* ;
- un extrait de *Diospyros mespiliformis*, un extrait de *Furcellaria lumbricalis* et un extrait de *Lampsana communis* ;
- un extrait de *Diospyros mespiliformis*, un extrait de Sanicle d'Europe, un extrait de *Furcellaria lumbricalis* et un extrait de *Lampsana communis* ;
pour lutter contre et/ou prévenir les effets de la pollution sur la peau, en particulier, pour protéger la peau contre les effets cutanés néfastes de la pollution atmosphérique et/ou réparer les désordres cutanés, c'est-à-dire les manifestations indésirables visibles sur la peau, induits par la pollution atmosphérique ; l'utilisation selon l'invention est avantageuse que la pollution atmosphérique résulte de la présence de polluants intérieurs ou extérieurs, il peut s'agir en particulier d'une pollution résultant de la présence dans l'air environnant d'agents oxydants tels que l'ozone ou les oxydes d'azote, de monoxyde de carbone, d'hydrocarbures et solvants, de dioxyde de soufre, de métaux, de formaldéhyde, de fumée de cigarette, de particules ou fines poussières en suspension, d'ondes électromagnétiques.

Plus particulièrement, l'utilisation d'une des associations selon l'invention ou d'une composition la comprenant permet de prévenir l'apparition des manifestations cutanées visibles sur la peau, signes de sa dégradation et/ou de réparer ces manifestations ou désordres cutanés induits par la pollution par les activités suivantes :
- augmentation de la cohésion cutanée et du *stratum corneum* et amélioration de la fonction barrière de la peau ;
- restauration de l'homéostasie des processus de prolifération et de différenciation des kératinocytes ;
- restauration de la matrice dermique ;
- restauration de l'hydratation cutanée ;
- amélioration de la cohésion des cellules cutanées ;
- amélioration de la communication cellulaire ;
- restauration de la jonction dermo-épidermique.

Sur la base de ces propriétés, l'utilisation d'une des associations selon l'invention ou d'une composition la comprenant est particulièrement utile pour prévenir et/ou limiter et/ou corriger le vieillissement de la peau induit par la pollution et les manifestations cutanées qui lui sont associées, en particulier, prévenir et/ou corriger les rides et ridules et/ou conserver l'éclat et/ou augmenter l'hydratation et/ou réguler la desquamation et/ou maintenir ou stimuler la souplesse de la peau soumise à la pollution.

La présente invention concerne enfin un procédé de soin cosmétique, pour prévenir et/ou réparer les effets de la pollution sur la peau, caractérisé en ce qu'on applique sur la peau une quantité adéquate d'un extrait de *Diospyros mespiliformis* seul ou en association avec un extrait de Sanicle d'Europe et/ou un extrait de *Furcellaria lumbricalis* et/ou un extrait de *Lampsona communis* ou d'une composition comprenant ledit extrait ou l'une desdites associations.

### Figures

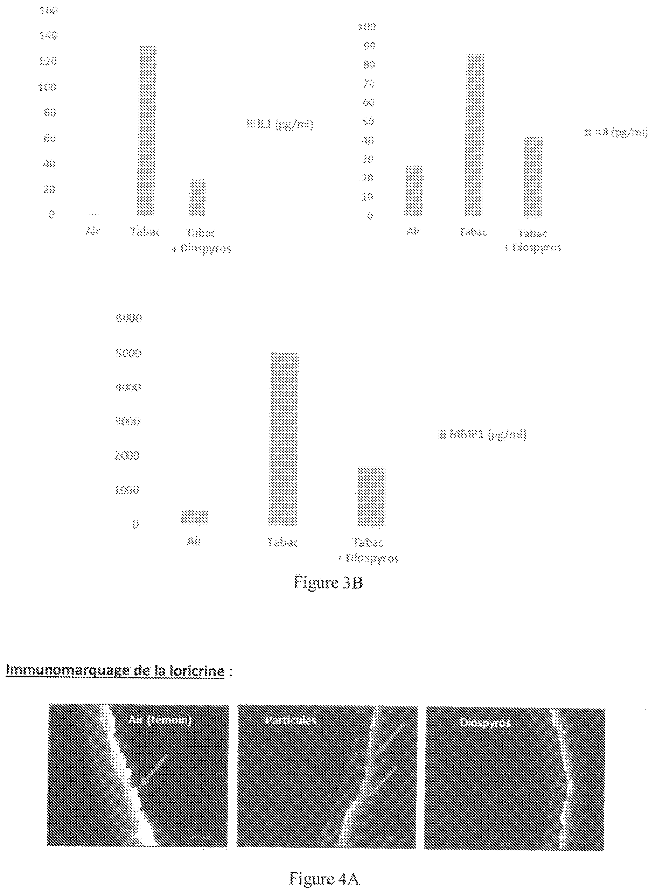

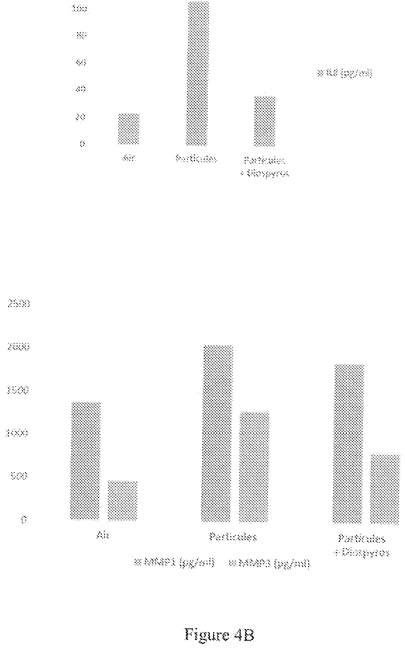

La Figure 1 représente le module d'exposition Vitrocell® utilisé dans le modèle formaldéhyde.
Les résultats des études protéomiques conduites sur un modèle d'épiderme traité avec un extrait de *Diospyros mespiliformis* exposé à du formaldéhyde sont présentés aux Figures 2A et 2B.
Les résultats des études protéomiques conduites sur un modèle d'épiderme traité avec un extrait de *Diospyros mespiliformis* exposé à de la fumée de tabac sont présentés aux Figures 3A et 3B.
Les résultats des études protéomiques conduites sur un modèle d'épiderme traité avec un extrait de *Diospyros mespiliformis* exposé à des particules fines sont présentés aux Figures 4A et 4B.
La Figure 5 est un histogramme représentant l'effet sur le taux de ROS mitochondriaux de kératinocytes exposés à différentes conditions (contrôle de cellules non traitées, H₂O₂, resvératrol et extrait de Sanicle à différentes concentrations).
La Figure 6 est un histogramme représentant l'effet sur le taux de ROS cellulaires de kératinocytes exposés à différentes conditions (contrôle de cellules non traitées, H₂O₂, resvératrol et extrait de Sanicle à différentes concentrations).
La Figure 7 est un histogramme représentant l'effet sur l'activité de la SOD cellulaire de kératinocytes exposés à différentes conditions (contrôle de cellules non traitées, H₂O₂, resvératrol et extrait de Sanicle à différentes concentrations).
La Figure 8 est un histogramme représentant l'effet sur l'activité de la SOD mitochondriale de kératinocytes exposés à différentes conditions (contrôle de cellules non traitées, H₂O₂, resvératrol et extrait de Sanicle à différentes concentrations).
La Figure 9 est un histogramme représentant l'effet sur le niveau de glutathion total de kératinocytes exposés à différentes conditions (contrôle de cellules non traitées, H₂O₂, resvératrol et extrait de Sanicle à différentes concentrations).
La Figure 10 est un histogramme représentant le taux d'hydratation cutanée d'explants de peau exposé à la pollution de gaz d'échappement et traité ou non à un extrait de *Furcellaria lumbricalis.*

### EXEMPLE 1

La mise en évidence de l'activité anti-pollution de l'extrait de *Diospyros mespiliformis* a été permise par la mise au point de nouveaux modèles capables de caractériser les effets de divers polluants de l'air intérieur et extérieur lors d'exposition unique ou répétée sur la réponse biologique d'épithélia reconstitués *in vitro.*

Brièvement, le modèle d'étude choisi est l'épiderme reconstruit RHE (SkinEthic Lyon, France). Il s'agit d'un épiderme reconstitué à partir de cellules primaires humaines. Les kératinocytes sont cultivés, en interface air/liquide, dans un milieu défini sans sérum sur des inserts ayant une membrane en polycarbonate (Insert Nunc 0.5cm²).

La mise en place d'un modèle d'exposition en interface air-liquide d'épidermes reconstruits permet d'étudier les interactions entre cellules épithéliales cutanées, dans un environnement proche de l'*in vivo*, et polluants environnementaux.

Trois types de polluants ont été appliqués à la surface de l'épiderme : le formaldéhyde, la fumée de tabac, les particules.

### Modèle formaldéhyde

Le formaldéhyde, mieux connu sous le nom de formol lorsqu'il est dissout dans l'eau, est un composé organique très volatil (COV) appartenant à la famille des aldéhydes. De faible poids moléculaire, cette substance a la propriété de devenir gazeuse à température ambiante et est actuellement fréquemment retrouvée dans les environnements intérieurs.

Le formaldéhyde a de très nombreuses sources parmi lesquelles : les sources de combustion englobant la fumée de tabac, les bougies, les bâtonnets d'encens, les cheminées à foyer ouvert ainsi que les cuisinières à gaz, les poêles à pétrole, les produits de construction et de décoration contenant des composants à base de formaldéhyde.

Pour l'étude, le modèle d'épiderme reconstruit a été exposé à une dose de formaldéhyde de 2100 ppm.

Les expositions sont réalisées dans un module d'exposition Vitrocell© six puits adapté aux échantillons. Ce module se compose de deux parties assurant une interface Air/Liquide avec un contact (i) de la partie basale des épidermes avec le milieu nutritif et (ii) de la partie apicale avec l'atmosphère générée (air ou polluant(s)) (**Figure 1**) :
- la partie A qui accueille les inserts (les épidermes). Le milieu de culture de maintien (MM) est déposé dans la partie inférieure du module thermostatée (37°C) grâce à une circulation d'eau.
- le contact direct entre atmosphère gazeuse et surface tissulaire est assuré par la partie B du module d'exposition et des trompettes d'admission du polluant.

Différents paramètres, comme la vitesse du flux d'air, le temps d'exposition des échantillons et la fréquence des expositions ont été définis avec une atmosphère « contrôle », comme l'air. L'objectif de cette étape a été de définir les meilleures conditions permettant des expositions en interface air/liquide sans qu'il y ait de souffrance tissulaire.

### Modèle fumée de tabac

La fumée de tabac est un mélange contenant des aldéhydes, dont le formaldéhyde et l'acétaldéhyde, et un grand nombre de composés organiques volatiles, a été générée à partir d'une cigarette commerciale, 10 x 20 ml ont été prélevés avec une seringue et introduit dans une chambre d'exposition. Les épidermes ont été exposés à 200 ml de fumée du tabac pendant 60 minutes à 37°C. Un "contrôle-air" a été étudié en parallèle.

### Modèle particules fines

Les particules fines proviennent du trafic routier, plus particulièrement des moteurs diésels des véhicules, mais également de l'industrie, de l'agriculture et de la combustion (cheminées, chauffage individuel...). Les microparticules, de la taille du micromètre, ne sont pas visibles à l'oeil nu. Ce sont celles qui sont mesurées dans l'air à travers les particules PM10 (taille inférieure à 10 µm, 6 à 8 fois plus petites que la taille d'une cellule) et les particules fines ou PM2,5 (taille inférieure à 2,5 µm, comme les bactéries).

Pour l'étude, les particules (composition : tableau ci-dessous) ont été appliquées par voie topique à la surface du tissu reconstruit. Les épidermes ont été exposés aux particules pendant 60 minutes à 37°C. Un "contrôle-air" a été étudié en parallèle.

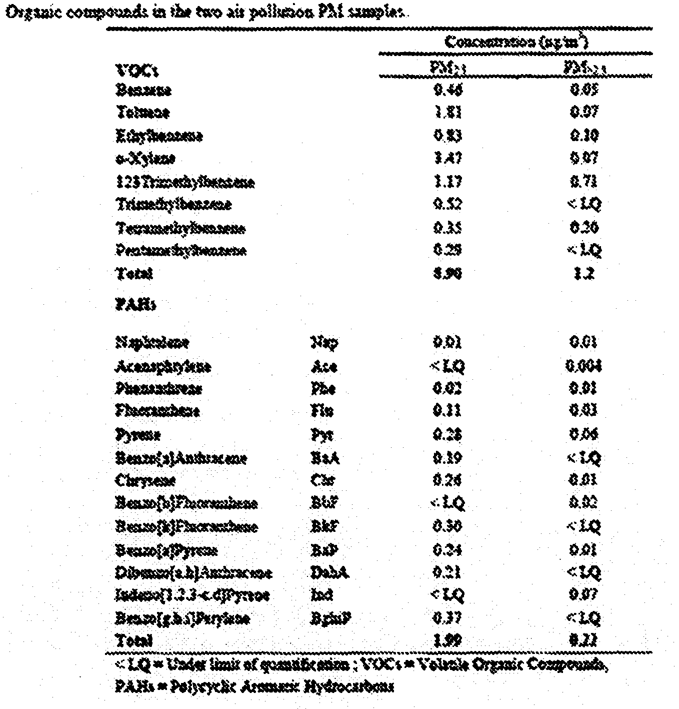

Diverses méthodes d'investigation ont permis de caractériser l'impact de ces polluants sur le modèle d'épiderme reconstruit Skinethic®:
- méthode transcriptomique réalisée par PCR quantitative (126 gènes « épidermiques ») ;
- méthode protéomique réalisée par immunohistochimie ;
- méthode de dosage des milieux de culture par la technique Elisa et la technique multiplex.

Dans le cadre de l'évaluation de l'effet protecteur de l'extrait de *Diospyros mespiliformis* vis-à-vis des modifications fonctionnelles et structurelles de l'épiderme induites par les polluants (formaldéhyde, fumée de cigarette, particules), les essais suivants ont été conduits :
L'épiderme reconstruit Skinethic® est traité à chacun des polluants associé ou non à un extrait *Diospyros mespiliformis* appliqué à la surface du tissu.
L'extrait de *Diospyros mespiliformis* testé est un extrait aqueux de feuilles stabilisé par de la maltodextrine obtenu par les étapes suivantes :
   - broyage de feuilles de *Diospyros mespiliformis* ;
   - extraction à l'eau ;
   - séparation ;
   - purification ;
   - ajout de maltodextrine ;
   - filtration ;
   - séchage par atomisation.

Caractérisation : poudre de couleur jaune-orange à beige avec une odeur caractéristique ; préparé en solution aqueuse à 1%, son pH est compris entre 5,0 et 7,0.

A l'issu des traitements, chaque épiderme est conservé pour réaliser des analyses de l'expression des gènes par RTqPCR et des immunomarquages de cibles épidermiques. Les milieux de culture sont utilisés pour les dosages par Elisa et par Multiplex.

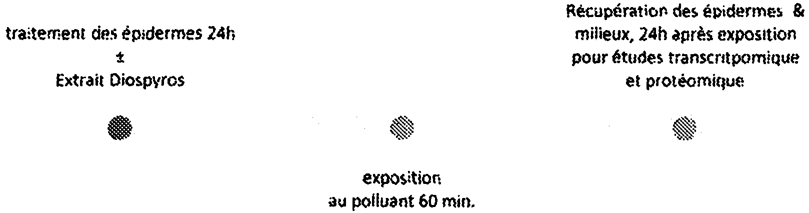

### ETUDE TRANSCRIPTOMIQUE :

L'étude transcriptomique est réalisée par PCR quantitative (qPCR) sur les épidermes reconstruits Skinethic®, permettant d'étudier l'expression de nombreux gènes. Les ARNs sont extraits en utilisant du tampon RLT puis un kit d'extraction RNeasy microkit de Qiagen ; ils sont ensuite dosés puis rétro-transcrits. La qPCR est alors réalisée sur des plaques 128 gènes des fonctions épidermiques (Roche Diagnostics).

L'analyse des résultats se fait par calcul de la différence d'expression entre deux conditions, appelée Fold Change (FC). On considère qu'une augmentation d'expression par rapport au témoin se traduit par un FC ≥ 1,5 ; et qu'une diminution d'expression par rapport au témoin se traduit par un FC ≤ 0,7.

### ETUDE PROTEOMIQUE :

L'étude protéomique est réalisée par immunohistochimie, permettant d'étudier la présence de nombreuses protéines.

Les épidermes reconstruits sont fixés dans de la formaline 10% pendant 72 h, puis déshydratés et inclus dans des blocs de paraffine, avant d'être coupés en sections de 5 µm. Les coupes sont déparaffinées puis démasquées (Abcam) avant d'être traitées avec 0,1% triton X-100 (Sigma) et Bovine Serum Albumine 2% (BSA - Sigma) pendant 10 minutes, afin de perméabiliser la membrane et saturer les sites de fixation.

Les coupes sont ensuite incubées pendant 1 h avec les anticorps primaires dirigés contre la loricrine et le 4-HNE (4-hydroxynonénal).

Les photographies sont prises à l'aide d'un microscope de conversion de fluorescence (Nikon Eclipse 50i).

### DOSAGE PAR ELISA, PAR MULTIPLEXAGE (TECHNOLOGIE LUMINEX) :

Le test ELISA (acronyme de *Enzyme Linked ImmunoSorbent Assay*) est un test immunologique destiné à détecter et/ou doser une protéine dans un liquide biologique. Dans la technique de dosage dite "en sandwich", les puits d'une microplaque sont tapissés avec un anticorps de capture capable de lier spécifiquement l'antigène recherché. Lors de cette opération appelée *coating*, l'anticorps de capture se fixe au plastique des puits par interaction électrostatique. Un deuxième anticorps, l'anticorps traceur, capable de se lier à l'antigène capturé est alors ajouté dans le puits et les anticorps traceurs non fixés sont éliminées par rinçage. L'anticorps traceur est couplé à une enzyme catalysant la formation d'un produit coloré. La réaction peut ainsi être quantifiée par colorimétrie à partir d'une courbe d'étalonnage réalisée avec des concentrations connues d'antigène puisque le nombre de molécules d'anticorps traceur fixées dépend du nombre de molécules d'antigènes immobilisées par l'anticorps de capture. Ce test est utilisé pour détecter la présence de IL-1α et de IL-8.

La technologie Luminex, basée sur le principe d'un ELISA combiné à de la cytométrie en flux, permet le dosage simultané de nombreuses cibles protéiques ou nucléiques dans de faibles volumes d'échantillons. La technologie fait appel à l'utilisation de microsphères qui sont soit des billes en polystyrène soit des billes magnétiques colorées par un mélange de deux fluorochromes dont le ratio est connu et parfaitement défini pour chaque type de billes.

Une fois les molécules d'intérêt fixées à la surface des billes, ces dernières sont incubées avec les échantillons à analyser. Après avoir suivi un protocole expérimental de type ELISA, les résultats sont obtenus par lecture sur un Luminex qui, grâce à un module de pipetage et de gestion des fluides, aspire les échantillons contenant les billes en suspension dans une veine liquide de manière à ce qu'elle soient les unes à la suite des autres (analogie avec la cytométrie de flux) puis excitées par un jeu de deux lasers :
- un laser rouge permettant de déterminer le code couleur de chaque bille,
- un laser vert excitant un fluorochrome couplé à une molécule reporter qui permet de détecter et de doser l'interaction antigène-anticorps.

Ce test est utilisé pour détecter la présence de MMP1, MM P3 et MMP9.

### RESULTATS

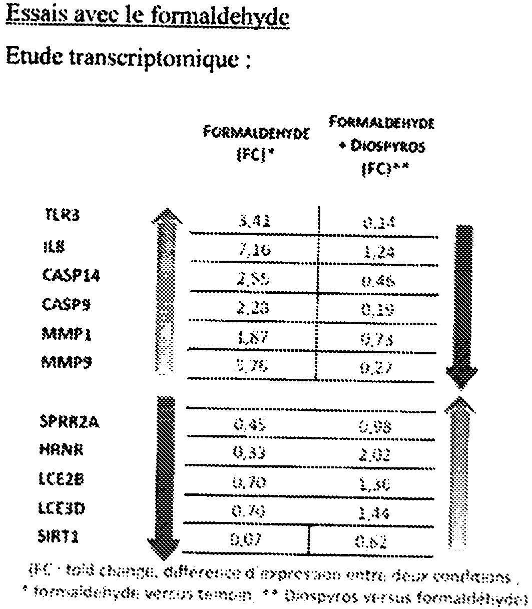

Les résultats des études protéomiques sont présentés aux **Figures 2A** et **2B**.

L'extrait de *Diospyros mespiliformis* exerce un effet protecteur de la barrière cutanée en augmentant l'expression des marqueurs constitutifs de l'enveloppe cornée (hornerine HRNR, Small Proline Rich Protein SPRR2A, LCE2B, LCE3D). Ces résultats se confirment avec l'identification par immunohistochimie de la loricrine, protéine majoritaire de l'enveloppe cornée. Egalement, l'expression de SIRT1 impliqué dans l'homéostasie des processus de prolifération et de différenciation des kératinocytes est sensiblement ré-augmentée.

L'extrait de *Diospyros mespiliformis* protège l'épiderme soumis au formaldéhyde en ne sollicitant plus les acteurs de l'inflammation. Les résultats montrent que les marqueurs de l'inflammation, de l'apoptose et de la dégradation matricielle sont sous-exprimés en présence d'extrait de *Diospyros mespiliformis* dans les conditions expérimentales choisies. Le dosage de la cytokine IL8 et des protéases matricielles MMP1, MMP9 confirment les résultats de l'étude transcriptomique.

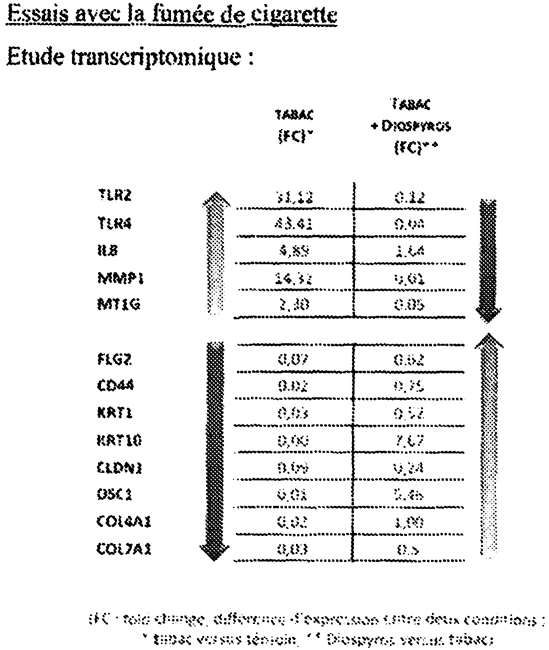

Les résultats des études protéomiques sont présentés aux **Figures 3A** et **3B**.

L'extrait de *Diospyros mespiliformis* protège l'épiderme soumis à la fumée de cigarette en ne sollicitant plus les acteurs de l'inflammation. Les résultats montrent que les marqueurs de l'inflammation, de l'apoptose et de la dégradation matricielle sont sous-exprimés en présence de l'extrait dans les conditions expérimentales choisies. Le dosage des cytokines IL1α, IL8 et de MMP1 confirment les résultats de l'étude transcriptomique.

L'extrait de *Diospyros mespiliformis* permet également d'assurer le maintien d'un bon niveau d'hydratation (FLG2, CD44).

Les résultats obtenus par traitement de l'épiderme avec l'extrait de *Diospyros mespiliformis* maintient une bonne conservation de la cohésion cellulaire et de la communication cellulaire assurées notamment par la claudine 1 (CLDN1) et la desmocoline 1 (DSC1).

L'extrait de *Diospyros mespiliformis* préserve la jonction dermo-épidermique des effets de la fumée de cigarette. L'expression des gènes des collagènes IV et VII est assurée.

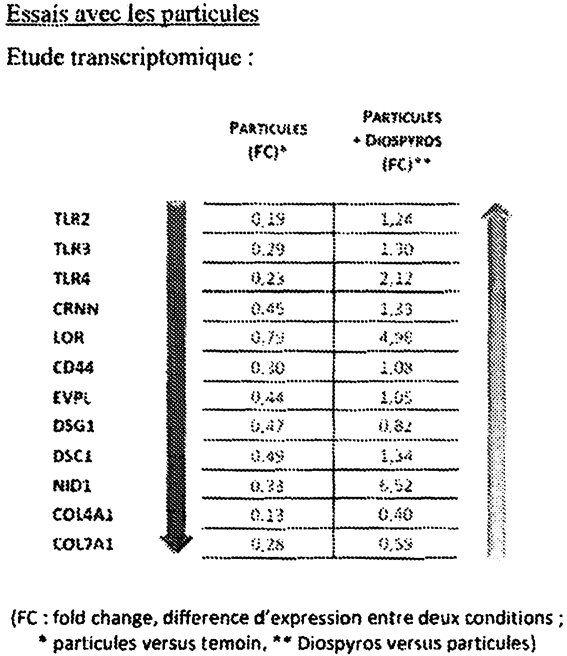

Les résultats des études protéomiques sont présentés aux **Figures 4A** et **4B**.

L'extrait de *Diospyros mespiliformis* protège la fonction barrière de la peau est protégée des particules. Notamment. l'expression de la loricrine au rôle clé dans la fonction barrière est fortement augmentée.

Les résultats obtenus par traitement de l'épiderme avec l'extrait de *Diospyros mespiliformis* montrent la conservation de la cohésion cellulaire et de la communication cellulaire assurées notamment par la claudine 1 (CLDN1) et la desmocolline 1 (DSC1).

L'extrait de *Diospyros mespiliformis* tend à protéger la jonction dermo-épidermique des effets des particules. L'expression des gènes des composants majoritaires de la JDE (collagènes IV, VII et nidogène I) est augmentée.

### EXEMPLE 2

L'objectif de cette étude est la caractérisation de la propriété antioxydante d'un extrait de Sanicle d'Europe en évaluant les paramètres de l'état redox après induction d'un stress oxydatif par le peroxyde d'hydrogène (H₂O₂) dans des cultures primaires de keratinocytes humains normaux.

L'extrait testé est un extrait hydro alcoolique obtenu comme suit :
- séchage des parties aériennes ;
- broyage ;
- extraction éthanol/eau (80:20) ;
- filtration ;
- concentration sous vide par évaporation des solvants d'extraction ;
- ajustement de la concentration (ajout de glycérol).

Caractérisation : liquide légèrement visqueux jaune-marron et d'odeur caractéristique. pH = 3.5 - 5.0 ; densité relative : 1.050-1.200 ; indice de réfraction : 1.370 - 1.400; rapport plante sèche/extrait = 1/10.

La technologie Mitosafe® permet d'explorer les effets antioxidants d'actifs au niveau mitochondrial. Cette technologie consiste en l'étude intégrée de fonctions mitochondriales dans des cellules vivantes par la mesure multiplexée de paramètres tels que viabilité cellulaire, production de radicaux libres oxygènes cellulaires et mitochondriaux, évaluation des défenses anti-oxydantes. Ces tests en plaque 96 puits sont réalisés sur primocultures de cellules épidermiques humaines.

Ont été mesurés:
- les espèces réactives de l'oxygène mitochondriales ;
- le taux de production cellulaire ROS ;
- la superoxyde dismutase mitochondriale et cellulaire (SOD) :
- le glutathion total (forme oxydée et réduite).

Dans les kératinocytes, l'exposition à l'H₂O₂ augmente la production des ROS mitochondriales ce qui entraine une sur-régulation de l'activité SOD mitochondriale et l'accumulation intracellulaire de ROS. Elle est également responsable de la hausse du taux de glutathion total, en réponse à l'augmentation intracellulaire des ROS. La régulation à la hausse de l'activité de la SOD mitochondriale et le niveau de glutathion sont des mécanismes compensatoires qui contrecarrent le stress oxydatif induit par l'accumulation de ROS dans l'espace intracellulaire. La SOD cellulaire est aussi un mécanisme direct antioxydant. Pourtant, dans ce modèle, il n'est pas induit par l'exposition à H₂O₂.

### Propriété anti-oxydante à travers la production des ROS cellulaires :

La méthodologie Mitoread AntiOx cROS consiste en la quantification de l'activité antioxydante des ingrédients vers les espèces réactives de l'oxygène cellulaires (ROS) à l'aide un marqueur fluorescent.

Tout d'abord, les cellules ont été pré-incubées avec la sonde fluorescente à 37°C pendant 30 minutes. Apres lavage, les cellules ont été co-incubées avec le composé de référence antioxydant. Trolox 10 µM ou les extraits à des concentrations déterminées non cytotoxiques en présence du composé de référence pro-oxydant, H₂O₂ 100 µM. La production de ROS cellulaires a été immédiatement enregistrée pendant 60 minutes. La fluorescence est enregistrée toutes les cinq minutes en utilisant un lecteur de microplaques (Varioskan-Thermo). Les cellules ont été maintenues à 37°C au cours de la cinétique. Tous les extraits ont été testés en quadruplicate. Leurs effets ont été comparés simultanément aux témoins négatif (H₂O₂) et positif (trolox).

### Propriété anti-oxydante à travers la production des ROS mitochondriales :

La méthodologie Mitoread AntiOx mtROS consiste en la quantification de l'activité antioxydante des ingrédients vers les espèces réactives de l'oxygène mitochondriales (ROS) à l'aide un marqueur fluorescent.

Les cellules ont été pré-incubées a 37°C pendant 60 minutes avec l'antioxydant de référence, le resveratrol à 1 µM, ou les principes actifs aux concentrations prédéterminés.

Ensuite, ils ont été incubés avec la sonde fluorescente à 37°C pendant 30 minutes, en remplacement des composés antioxydants. Apres lavage, les cellules ont été traitées avec le composé pro-oxydant de référence, le peroxyde d'hydrogène H₂O₂ à 100 µM. La production de ROS mitochondriales a été immédiatement enregistrée pendant 60 minutes. La fluorescence est enregistrée toutes les cinq minutes en utilisant un lecteur de microplaques (Varioskan-Thermo). Les cellules ont été maintenues à 37°C au cours de la cinétique. Tous les extraits ont été testés en quadruplicate. Leurs effets ont été comparés simultanément aux témoins négatif (H₂O₂) et positif (resveratrol).

### Propriété anti-oxydante à travers les activités SuperOxyde Dismutaste cellulaire et mitochondriale :

La méthodologie Mitoread RedOx c/mtSOD consiste en la quantification des activités SOD cellulaire et mitochondriale.

Les cellules ont été pré-incubées a 37°C pendant 60 minutes avec l'antioxydant de référence, le resveratrol à 1 µM, ou les principes actifs aux concentrations prédéterminées. Les cellules ont été traitées avec le compose pro-oxydant de référence, le peroxyde d'hydrogène H₂O₂ à 100 µM pendant 60 minutes. Immédiatement après, les extraits cellulaires ont été préparés et conservés à -80°C. Les activités enzymatiques ont été mesurées par une méthode fluorométrique et enzymatique en utilisant un lecteur de microplaques (Varioskan-Thermo). Tous les extraits ont été testés en triplicate. Leurs effets ont été comparés simultanément aux témoins négatif (H₂O₂) et positif (resveratrol).

### Propriété anti-oxydante à travers le taux de Glutathion total :

La méthodologie Mitoread RedOx GSHtot consiste en la quantification du taux de Glutathion total (GSH+GSSG).

Les cellules ont été pré-incubées à 37°C pendant 60 minutes avec l'antioxydant de référence, le resveratrol à 1 µM, ou l'extrait aux concentrations prédéterminées. Les cellules ont été traitées avec le composé pro-oxydant de référence, le peroxyde d'hydrogène H₂O₂ à 100 µM pendant 60 minutes. Immédiatement après, les extraits cellulaires ont été préparés et conservés à -80°C. Le taux de Glutathion total a été mesuré par une méthode fluorométrique et enzymatique en utilisant un lecteur de microplaques (Varioskan-Thermo).

Tous les extraits ont été testés en triplicata. Leurs effets ont été comparés simultanément aux témoins négatif (H₂O₂) et positif (resveratrol).

### Résultats

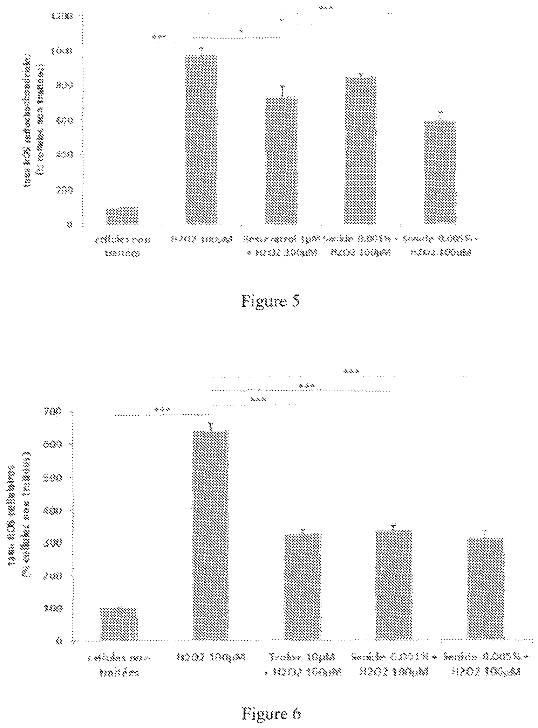

Les effets des contrôles négatifs et positifs ont été validés. 100 µM de H₂O₂ induit une importante augmentation d'un facteur 9,4 dans la production mitochondriale de ROS par rapport aux cellules non traitées. Le resveratrol à 1 µM réduit considérablement la surproduction de ROS mitochondriales induites par H₂O₂ dans les kératinocytes (près de 1,3 fois (p = 0,013)) (**Figure 5**).

L'extrait de sanicle réduit significativement le taux de ROS mitochondriales induites par H₂O₂ démontrant une **activité antioxydante efficace** (**13%** à 0.001%, p<0,05 et **38%** à 0,005%, p<0,001).

Les effets des contrôles négatifs et positifs ont été validés. 100 µM de H₂O₂ induit une importante augmentation de 7,4 fois le taux de production de ROS cellulaires par rapport aux cellules non traitées. Le trolox à 10 µM réduit considérablement la surproduction de ROS cellulaires induites par H₂O₂ dans les kératinocytes (1,7 fois, p < 0,001).

Aux deux doses, l'extrait de sanicle réduit significativement la production des ROS cellulaires induites par H₂O₂ (**49%** en moyenne, p<0,001) (**Figure 6**)

Dans les kératinocytes humains, l'exposition au H₂O₂ a induit une tendance non significative à la diminution de l'activité SOD cellulaire de près de 30% (p = 0,075) par rapport aux cellules non-traitées alors qu'elle a augmenté de manière significative l'activité de la SOD mitochondriale de près de 211% (p = 0,006) par rapport aux cellules non traitées.

Le composé de référence antioxydant, le resveratrol à 1 µM, a augmenté de manière significative l'activité SOD cellulaire de près de 40% (p = 0,036) par rapport aux cellules non traitées en réponse à l'exposition à H₂O₂. En outre, le resveratrol à 1 µM a réduit significativement l'activité de la SOD mitochondriale induite par l'H₂O₂, par près de 66% (p = 0,021), maintenant un niveau mtSOD comparable à celle des cellules non traitées.

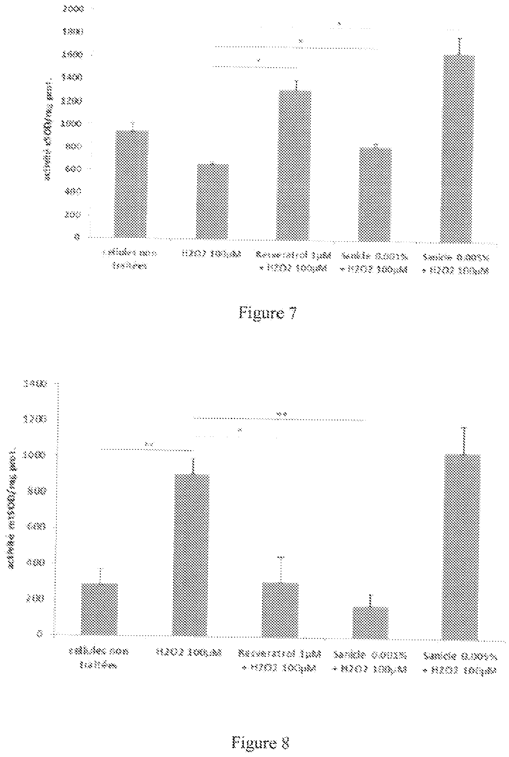

Le traitement des kératinocytes par l'extrait de sanicle à 0,001% conduit à une activité SOD cellulaire comparable à celle des cellules non traitées. A 0,005%, l'extrait de sanicle a augmenté l'activité SOD cellulaire sur celle induite par H₂O₂ par rapport aux cellules non traitées par 76% (p = 0,012), révélant une régulation à la hausse des défenses anti-oxydantes cellulaires en réponse à un stress oxydatif cellulaire **(****Figure 7**).

L'extrait de sanicle à 0,001% a restauré l'activité de la SOD mitochondriale comparable à celle des cellules non traitées, diminuant considérablement son induction par H₂O₂ de près de 80% (p = 0,008), de manière similaire à l'effet du resveratrol à 1 µM.

L'extrait de sanicle à 0,005% a maintenu l'activité SOD mitochondriale induite par H₂O₂ **(****Figure 8**).

H₂O₂ à 100 µM a augmenté de façon significative le niveau de glutathion total de près de 2,9 fois par rapport aux cellules non traitées (p = 0,044), révélant une régulation a la hausse du glutathion en réponse au stress oxydatif induit par H₂O₂. La pré-incubation avec le resveratrol à 1 µM a réduit significativement le niveau de glutathion total après traitement par H₂O₂ (94%), restaurant un niveau comparable à celui des cellules non-traitées (p = 0,006).

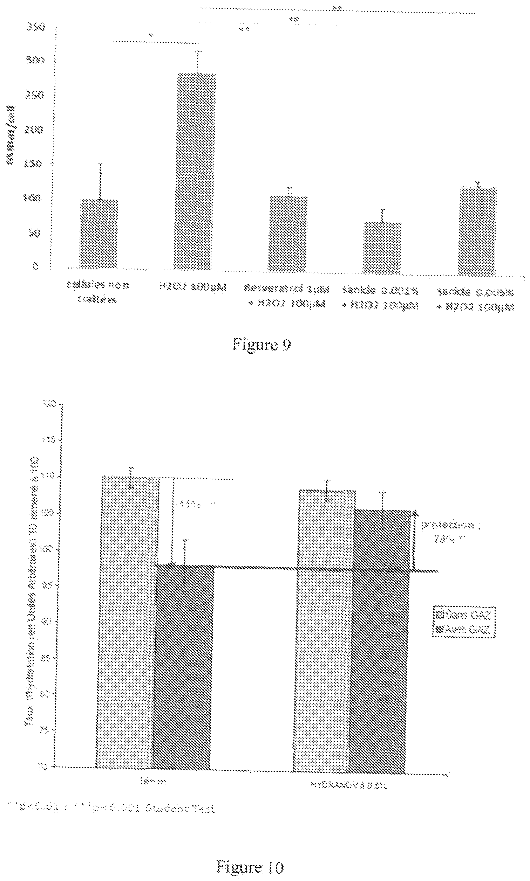

L'extrait de sanicle a diminué de manière significative (comme le Resveratrol à 1µM), le niveau du glutathion total après traitement au H₂O₂ au niveau comparable à celui des cellules non traitées **(****Figure 9**).

### Conclusion

L'exposition au H₂O₂ a été identifiée comme un inducteur de dommages cellulaires, compromettant l'équilibre d'oxydoréduction dans les kératinocytes humains primaires. Les effets de l'H₂O₂ (100 µM, 1 heure) dans les kératinocytes humains sont résumés dans le tableau ci-dessous.

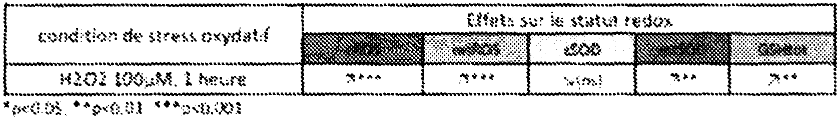

Dans les conditions expérimentales, H₂O₂ a généré des ROS mitochondriales, conduisant à l'accumulation des ROS intracellulaires. L'augmentation de l'activité de la SOD mitochondriale et l'augmentation du niveau de glutathion total sont une réponse au stress oxydatif induit par H₂O₂ pour contrer augmentation précoce de la production en ROS.

L'augmentation de la SOD mitochondriale et de la GSH peuvent être directement responsable de la réduction observée de la SOD cellulaire, à travers une boucle rétroactive, expliquant la diminution de son activité où l'H₂O₂ exogène apporté par le traitement surmonte l'activité de la SOD cellulaire, qui convertit O₂ en H₂O₂.

Les effets de l'extrait de sanicle combinés à une exposition à H₂O₂ sont résumés dans le tableau ci-dessous.

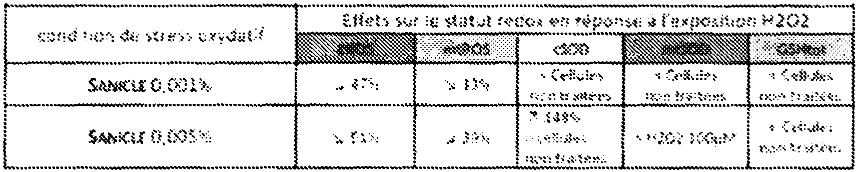

L'extrait de sanicle à 0,001% est un antioxydant efficace vis-à-vis des ROS mitochondriales et cellulaires induites par H₂O₂. De ce fait, l'extrait de sanicle diminue la nécessité d'augmenter les enzymes anti-oxydantes SOD et le glutathion, système antioxydant non enzymatique.

L'extrait de sanicle à 0.005% rehausse l'activité de la SOD cellulaire, déjà sollicitée en présence de H₂O₂, potentialisant les premières défenses anti-oxydantes.

### EXEMPLE 3

L'objectif de cette étude est de présenter l'effet anti-pollution d'un extrait de *Furcellaria lumbricalis* à la concentration de 0,5%.

Pour cela, il a été choisi la pollution due aux gaz d'échappement automobile contenant des éléments de pollution communs à de nombreux stress polluants comme la fumée de cigarette, CO, NO et NO₂, SO₂, mais aussi le benzène, les aldéhydes, les particules, les composés organiques volatiles (COV), les polychlorobiphényles (PCB), les métaux lourds,... (Dupuis.E., 2006).

L'effet de cette pollution a été mesuré sur des explants de peau humaine exposés à la pollution.

### MATERIEL ET METHODE

### Modèle biologique

Explants de peau humaine de 12 mm de diamètre, recueillis après une opération de plastic abdominale sur une femme caucasienne de 58 ans (Biopredic International, Rennes).

### Produits à l'essai et de référence

L'extrait testé est le produit commercial Hydranov P de Codif dont on rappelle qu'il s'agit d'un extrait l'oligofurcellarane enrichi en sels marins obtenu par dépolymérisation d'un furcellarane sulfaté (galactose et anhydrogalactose) puis réhydraté avec une solution enrichie en sels marins.

L'extrait ainsi obtenu a été dilué à 0,5% dans de l'eau.

### Système d'essai

Après réception des explants, ceux-ci sont stabilisés 16h à température ambiante. Suite à cette stabilisation. une mesure de l'hydratation est effectuée avant application des produits (T0). Les fragments de peau sont ensuite traités par une solution d'un extrait de *Farcellaria lumbricalis* à 0,5% pendant 30min. Ces explants sont ensuite exposés aux gaz d'échappement automobile pendant 5h. Ils sont ensuite incubés à l'interface air/milieu dans un milieu long terme tamponné (Hepes) pendant 18h à 20-22°C. Après 18h, une mesure de l'hydratation est effectuée (Tl). La manipulation réalisée sur ces explants est schématisée ci-dessous :

### Traitement par le gaz

Le gaz utilisé dans ce test provient d'un véhicule diesel (1989) de marque Renault sans filtre à particules et est collecté en utilisant un sac standard airtight (brand SKC, réf. Cat. No 236-005).

A titre indicatif, la composition du gaz est la suivante :

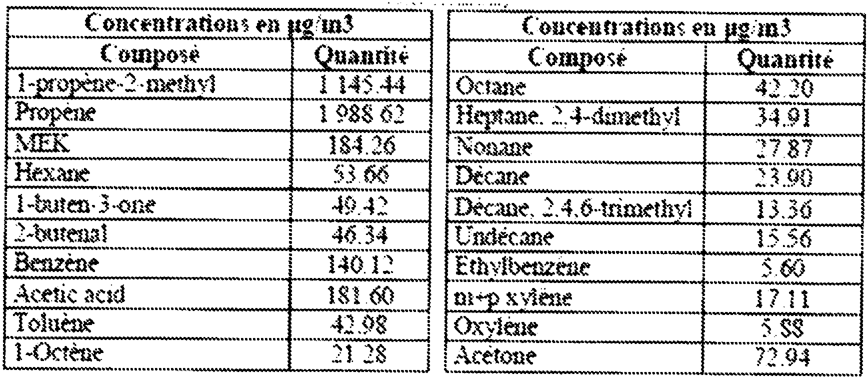

### Mesure de l'hydratation

### a. Principe

Le taux d'hydratation cutanée a été étudié au moyen d'un Cornéomètre® CM 825 MDD (COURAGE & KHAZAKA). Cet appareil permet de déterminer le degré d'humidité des couches cutanées les plus externes du *stratum corneum.* Le principe d'action du Cornéomètre repose sur la possibilité pour le détecteur, conçu en condensateur, de modifier sa capacité. La face de la tête de mesure, en contact avec la peau, modifie sa capacité selon le degré d'humidité de la peau. Ces modifications sont directement transcrites sur informatique.

Différentes méthodes existent pour mesurer le taux d'hydratation cutanée. Parmi elles, la cornéométrie est une méthode classique en cosmétologie. Basée sur la possibilité pour le détecteur de modifier sa capacité en fonction du degré d'humidité des couches cutanées les plus superficielles du *stratum corneum,* le Cornéomètre permet donc de quantifier, *in vivo* et en direct sur l'Homme, l'effet de produits cosmétiques ou dermopharmaceutiques sur le taux d'hydratation du *stratum corneum.*

### b. Paramètre étudié

Le taux d'hydratation est exprimé en unités arbitraires.

**Une augmentation des valeurs d'hydratation cutanées traduit un effet hydratant du produit.**

### Analyse statistique

Les données brutes sont transférées et traitées en utilisant le logiciel Excel. Le test utilisé est le test t de Student sur données appariées. Une différence entre 2 groupes est considérée comme statistiquement significatif si le p-value est inférieur à 0.05. et noté *p<0.05.

### RESULTATS

Dans les conditions du test, l'exposition aux gaz d'échappement automobiles provoque une nette diminution de l'hydratation (Figure 1 : Témoin + gaz par rapport au Témoin - gaz).

L'extrait de *Furcellaria lumbricalis* a fortement protégé la peau contre la chute du taux d'hydratation (+78%) dans les explants de peau humaine induite par la pollution.

Ces résultats sont illustrés à la **Figure 10****.**

### CONCLUSION

L'exposition à la pollution cause une chute du taux d'hydratation de la peau.

Au niveau des explants de peau humaine, l'extrait de *Furcellaria lumbricalis* à 0,5% protège contre la perte d'hydratation induite par la pollution.

### EXEMPLES DE COMPOSITIONS SELON L'INVENTION

**CREME ANTI-RIDES**

| | % |
|---|---|
| CARBOMER | 0,30 |
| GLYCERIN | 2,00 |
| DIMETHICONE | 3,00 |
| ISONONYL ISONONANOATE | 8,00 |
| CETEARYL ALCOHOL & CETEARYL GLUCOSIDE | 3,50 |
| TOCOPH EROL | 0,06 |
| GLYCERYL STEARATE & PEG-100 STEARATE | 2,50 |
| DICAPRYLYL CARBONATE | 4,00 |
| SODIUM HYDROXIDE | 0,08 |
| TAPIOCA STARCH | 1,50 |
| BIOSACCHARIDE GUM-4 | 1,00 |
| LAMPSANE | 0,50 |
| FURCELLARIA LUMBRICALIS | 1,00 |
| DIOSPYROS MESPILIFORMIS | 0,50 |
| SANICULA EUROPAEA | 0,50 |
| CONSERVATEURS | Q.S. |
| DISODIUM EDTA | 0,10 |
| PARFUM | Q.S. |
| EAU DEMINERALISEE | Q.S.P 100 |

**SERUM**

| | % |
|---|---|
| CETEARYL ETHYLHEXANOATE | 2.50 |
| STEARYL HEPTANOATE | 1,50 |
| CAPRYLIC/CAPRIC TRIGLYCERIDE | 3,00 |
| PEG-20 METHYL GLUCOSE SESQUISTEARATE | 2.00 |
| DIMETHICONE | 1,50 |
| TOCOPHEROL | 0,02 |
| ACRYLATES/C10-30 ALKYL ACRYLATE CROSSPOLYMER. | 0,25 |
| ISONONYL ISONONANOATE | 3,00 |
| C12-15 ALKYL BENZOATE | 2,50 |
| HYDROXYETHYLCELLULOSE | 0,10 |
| GLYCERIN | 5,00 |
| BUTYLENE GLYCOL | 2,00 |
| ETHYLHEXYLGLYCERIN | 0,20 |
| DISODIUM EDTA | 0,05 |
| TROMETHAMINE | 0,30 |
| LAMPSANE | 0,50 |
| FURCELLARIA LUMBRICALIS | 1,00 |
| DIOSPYROS MESPILIFORMIS | 0,50 |
| SANICU LA EUROPAEA | 0,50 |
| CONSERVATEURS | Q.S. |
| PARFUM | Q.s. |
| EAU DEMINERALISEE | Q.S.P 100 |

**EMULSION H/E**

| | % |
|---|---|
| CETEARYL ALCOHOL & CETEARYL GLUCOSIDE | 3,50 |
| GLYCERYL STEARATE & PEG-100 STEARATE | 2,00 |
| STEARETH-21 | 0,50 |
| HYDROGENATED COCO-GLYCERIDES | 2,00 |
| BEURRE DE KARITE | 1,00 |
| CYCLOM ETHI CONE | 6,00 |
| BHT | 0,02 |
| CARBOMER | 0,20 |
| GLYCERIN | 5,00 |
| SODIUM HYDROXIDE | 0,04 |
| SILICA | 1,40 |
| TOCOPHERYL ACETATE | 0,20 |
| DIMETHICONE & DIMETHICONOL | 0.50 |
| LAMPSANE | 0,50 |
| FURCELLARIA LUMBRICALIS | 1,00 |
| DIOSPYROS MESPILIFORMIS | 0,50 |
| SANICULA EUROPAEA | 0,50 |
| CONSERVATEURS | Q.S. |
| TETRASODIUM EDTA | 0,05 |
| PARFUM | Q.S. |
| EAU DEMINERALISEE | Q.S.P 100 |

**CREME JOUR SPF 20**

| | % |
|---|---|
| ETHYLHEXYLGLYCERIN | 0,20 |
| DISODIUM EDTA | 0,10 |
| XANTHAN GUM | 0,20 |
| GLYCOLS | 5,00 |
| ACACIA SENEGAL GUM | 0,01 |
| BUTYLOCTYL SALICYLATE | 5,00 |
| SODIUM POLYACRYLATE | 0,50 |
| MICA | 0,10 |
| POTASSIUM CETYL PHOSPHATE | 0,50 |
| BUTYL METHOXYDIBENZOYLMETHANE | 3,00 |
| OCTOCRYLENE | 2,70 |
| HOMOSALATE | 10,00 |
| CETEARYL ALCOHOL & CETEARYL GLUCOSIDE | 3,50 |
| C12-15 ALKYL BENZOATE | 8,00 |
| TAPIOCA STARCH | 2,00 |
| LAMPSANE | 0,50 |
| FURCELLARIA LUMBRICALIS | 1,00 |
| DIOSPYROS MESPILIFORMIS | 0,50 |
| SANICULA EUROPAEA | 0,50 |
| ALCOHOL | 3,00 |
| CONSERVATEURS | Q.S. |
| PARFUM | Q.S. |
| EAU DEMINERALISEE | Q.S.P 100 |

**EMULSION E/H**

| | % |
|---|---|
| PEG 30 DIPOLYHYDROXYSTEARATE | 4,00 |
| TRIGLYCERIDES C16-C18 HYDROGENEES | 5,00 |
| PEG-45 / DODECYL GLYCOL COPOLYMER | 1.20 |
| TRIGLYCERIDES C8C10 | 19,00 |
| HUILE DE VASELINE FLUIDE | 8,00 |
| GLYCOLS | 10,00 |
| LAMPSANE | 0,50 |
| FURCELLARIA LUMBRICALIS | 1,00 |
| DIOSPYROS MESPILIFORMIS | 0,50 |
| SANICULA EUROPAEA | 0,50 |
| CONSERVATEURS | Q.S. |
| PARFUM | Q.S. |
| EAU DEMINERALISEE | Q.S.P 100 |

**LOTION HYDROALCOOLIQUE**

| | % |
|---|---|
| GLYCOLS | 2,00 |
| TAMPON | 0,40 |
| CHLORURE DE SODIUM | 1,00 |
| ETHANOL | 5,00 |
| LAMPSANE | 0,50 |
| FURCELLARIA LUMBRICALIS | 1,00 |
| DIOSPYROS MESPILIFORMIS | 0,50 |
| SANICULA EUROPAEA | 0.50 |
| TROMETHAMINE | 0,80 |
| CONSERVATEURS | Q.S. |
| SOLUBILISANT | Q.S. |
| PARFUM | Q.S. |
| EAU DEMINERALISEE | Q.S.P 100 |

## Revendications

1. Utilisation cosmétique d'un extrait de *Diospyros mespiliformis* ou d'une composition le comprenant pour protéger la peau contre les effets cutanés néfastes de la pollution atmosphérique et/ou réparer les manifestations cutanées induites par la pollution atmosphérique

2. Utilisation selon la revendication 1, **caractérisée en ce que** la pollution atmosphérique résulte de la présence de polluants intérieurs ou extérieurs choisis parmi les agents oxydants, le monoxyde de carbone, les hydrocarbures et solvants, le dioxyde de soufre, les métaux, le formaldéhyde, la fumée de cigarette, les particules ou fines poussières en suspension et les ondes électromagnétiques.

3. Utilisation selon l'une quelconque des revendications précédentes, pour prévenir la dégradation et/ou de restaurer les désordres cutanés induits par la pollution par les activités suivantes :
- augmentation de la cohésion cutanée et du *stratum corneum* et amélioration de la fonction barrière de la peau ;
- restauration de l'homéostasie des processus de prolifération et de différenciation des kératinocytes ;
- restauration de la matrice dermique ;
- prévention et restauration de l'hydratation cutanée ;
- amélioration de la cohésion des cellules cutanées ;
- amélioration de la communication cellulaire ;
- restauration de la jonction dermo-épidermique.

4. Utilisation selon l'une quelconque des revendications précédentes, pour prévenir et/ou corriger le vieillissement de la peau induit par la pollution, en particulier, prévenir ou corriger les rides et ridules et/ou conserver l'éclat et/ou augmenter l'hydratation et/ou réguler la desquamation, maintenir ou stimuler la souplesse de la peau soumise à la pollution.

5. Utilisation selon l'une quelconque des revendications précédentes, **caractérisée en ce que** l'extrait est obtenu par broyage et extraction par solvants aqueux et/ou organiques d'une partie aérienne de la plante.

6. Utilisation selon l'une quelconque des revendications précédentes, **caractérisée en ce que** la composition comprend entre 0,01 et 10% d'extrait en poids.

7. Utilisation selon l'une quelconque des revendications précédentes, **caractérisée en ce que** la composition comprend des adoucissants, des colorants, des actifs filmogènes, des tensioactifs, des parfums, des conservateurs, des émulsionnants, des huiles, des glycols, des vitamines comme la vitamine E, des filtres UV.

8. Utilisation selon l'une quelconque des revendications précédentes, **caractérisée en ce que** la composition se présente sous la forme d'un gel, d'une lotion, d'une crème, d'une émulsion, d'un lait, d'un spray.

9. Utilisation selon l'une quelconque des revendications précédentes, **caractérisée en ce que** ledit extrait est associé à un extrait de Sanicle d'Europe.

10. Utilisation selon la revendication 9, **caractérisée en ce que** ledit extrait de Sanicle d'Europe est un extrait hydrosoluble des parties aériennes de la plante.

11. Utilisation selon l'une quelconque des revendications précédentes, **caractérisée en ce que** ledit extrait ou l'association d'extraits comprend en outre un extrait de *Furcellaria lumbricalis.*

12. Utilisation selon la revendication 11, **caractérisée en ce que** ledit extrait de *Furcellaria lumbricalis* comprend de l'oligofurcellarane enrichi en sels marins.

13. Utilisation selon l'une quelconque des revendications précédentes, **caractérisée en ce que** ledit extrait ou l'association d'extraits comprend en outre un extrait de *Lampsana communis.*

14. Utilisation selon la revendication 13, **caractérisée en ce que** ledit extrait de *Lampsana communis* est obtenu par extraction hydro-glycolique de la partie aérienne de la plante.

15. Procédé de soin cosmétique, pour lutter et prévenir les effets de la pollution sur la peau, **caractérisé en ce qu'**on applique sur la peau une quantité adéquate d'un extrait de *Diospyros mespiliformis* seul ou en association avec un extrait de Sanicle d'Europe et/un extrait de *Furcellaria lumbricalis* et/ou un extrait de *Lampsana communis* ou d'une composition comprenant ledit extrait ou l'une desdites associations.

## Patentansprüche

1. Kosmetische Verwendung eines *Diospyros-Mespiliformis-*Extrakts oder einer Zusammensetzung, die diesen umfasst, um die Haut gegen die schädlichen dermalen Effekte der atmosphärischen Verschmutzung zu schützen und/oder die dermalen Anzeichen zu beseitigen, die durch die atmosphärische Verschmutzung verursacht wurden.

2. Verwendung nach Anspruch 1, **dadurch gekennzeichnet, dass** sich die atmosphärische Verschmutzung aus der Anwesenheit von inneren oder äußeren Schmutzstoffen ergibt, ausgewählt aus den Oxidationsmitteln, Kohlenmonoxid, Kohlenwasserstoffen und Lösemitteln, Schwefeldioxid, Metallen, Formaldehyd, Zigarettenrauch, Partikeln oder Feinstäuben in Suspension und elektromagnetischen Wellen.

3. Verwendung nach einem der vorstehenden Ansprüche, um dem Abbau vorzubeugen und/oder die dermalen Störungen, die von der Verschmutzung verursacht wurden durch die folgenden Aktivitäten wiederherzustellen:
- Erhöhen der dermalen Bindung und des *Stratum corneum* und Verbessern der Barrierefunktion der Haut;
- Wiederherstellen der Homöostase der Ausbreitungs- und Differenzierungsprozesse der Keratinozyten;
- Wiederherstellen der Hautmatrix;
- Vorbeugen und Wiederherstellen der dermalen Hydratation;
- Verbessern der Bindung der dermalen Zellen;
- Verbessern der Zellkommunikation;
- Widerherstellen der dermo-epidermalen Verbindung.

4. Verwendung nach einem der vorstehenden Ansprüche, um der Alterung der Haut, die durch die Verschmutzung verursacht wurde, vorzubeugen und/oder sie zu korrigieren, insbesondere, um den Falten und Fältchen vorzubeugen oder sie zu korrigieren, und/oder den Glanz beizubehalten und/oder die Hydratation zu erhöhen und/oder die Abschuppung zu regeln, die Geschmeidigkeit der Haut, die der Verschmutzung ausgesetzt wurde, beizubehalten oder zu stimulieren.

5. Verwendung nach einem der vorstehenden Ansprüche, **dadurch gekennzeichnet, dass** der Extrakt durch Zerkleinern und Extrahieren durch wässrige und/oder organische Lösemittel eines oberirdischen Teils der Pflanze erhalten wird.

6. Verwendung nach einem der vorstehenden Ansprüche, **dadurch gekennzeichnet, dass** die Zusammensetzung zwischen 0,01 und 10 Gew.-% an Extrakt umfasst.

7. Verwendung nach einem der vorstehenden Ansprüche, **dadurch gekennzeichnet, dass** die Zusammensetzung Weichmacher, Farbstoffe, filmbildende Wirkstoffe, Tenside, Parfums, Konservierungsstoffe, Emulsionsmittel, Öle, Glykole, Vitamine, wie Vitamin E, UV-Filter umfasst.

8. Verwendung nach einem der vorstehenden Ansprüche, **dadurch gekennzeichnet, dass** sich die Zusammensetzung in Form eines Gels, einer Lotion, einer Creme, einer Emulsion, einer Milch, eines Sprays präsentiert.

9. Verwendung nach einem der vorstehenden Ansprüche, **dadurch gekennzeichnet, dass** der Extrakt mit einem Extrakt aus Waldsanikel assoziiert ist.

10. Verwendung nach Anspruch 9, **dadurch gekennzeichnet, dass** der Extrakt aus Waldsanikel ein wasserlöslicher Extrakt der überirdischen Teile der Pflanze ist.

11. Verwendung nach einem der vorstehenden Ansprüche, **dadurch gekennzeichnet, dass** der Extrakt oder die Assoziation von Extrakten weiter einen aus *Furcellaria-lumbricalis-*Extrakt umfasst.

12. Verwendung nach Anspruch 11, **dadurch gekennzeichnet, dass** der *Furcellaria-lumbricalis-*Extrakt mit Meersalzen angereichertes Oligofurcellaran umfasst.

13. Verwendung nach einem der vorstehenden Ansprüche, **dadurch gekennzeichnet, dass** der Extrakt oder die Assoziation von Extrakten weiter einen *Lampsana-communis-*Extrakt umfasst.

14. Verwendung nach Anspruch 13, **dadurch gekennzeichnet, dass** der *Lampsana-communis-*Extrakt durch Extrahieren von Hydro-Glykol des überirdischen Teils der Pflanze erhalten wird.

15. Verfahren zur kosmetischen Pflege, um die Effekte der Verschmutzung auf der Haut zu bekämpfen und ihnen vorzubeugen, **dadurch gekennzeichnet, dass** auf die Haut eine angemessene Menge eines *Diospyrosmespiliformis*-Extrakts alleine oder in Assoziation mit einem Waldsanikel-Extrakt und/oder einem *Furcellaria-lumbricalis-*Extrakt und/oder einem *Lampsana-communis*-Extrakt oder eine Zusammensetzung, den Extrakt oder eine der Assoziationen umfassend, aufgebracht wird.

## Claims

1. Cosmetic use of an extract of *Diospyros mespiliformis* or of a composition comprising it to protect the skin against the harmful cutaneous effects of atmospheric pollution and/or to repair the cutaneous manifestations induced by atmospheric pollution.

2. Use according to claim 1, **characterised in that** the atmospheric pollution results from the presence of indoor or outdoor pollutants selected from among oxidising agents, carbon monoxide, hydrocarbons and solvents, sulphur dioxide, metals, formaldehyde, cigarette smoke, particles or fine dust in suspension and electromagnetic waves.

3. Use according to any one of the preceding claims, to prevent the degradation and/or restore the skin disorders induced by pollution by the following activities:
- increase in the cutaneous cohesion and of the *stratum corneum* and improvement in the barrier function of the skin;
- restoration of the homeostasis of the proliferation and differentiation processes of keratinocytes;
- restoration of the dermal matrix;
- prevention and restoration of cutaneous hydration;
- improvement in the cohesion of cutaneous cells;
- improvement in cellular communication;
- restoration of the dermoepidermal junction.

4. Use according to any one of the preceding claims, to prevent and/or correct the ageing of the skin induced by pollution, in particular, prevent or correct wrinkles and fine lines and/or retain shine and/or increase hydration and/or regulate flaking, maintain or stimulate the flexibility of the skin subjected to pollution.

5. Use according to any one of the preceding claims, **characterised in that** the extract is obtained by grinding and extracting via aqueous and/or organic solvents of an aerial part of the plant.

6. Use according to any one of the preceding claims, **characterised in that** the composition comprises between 0.01 and 10% of extract by weight.

7. Use according to any one of the preceding claims, **characterised in that** the composition comprises softeners, colourants, film-forming agents, surfactants, perfumes, preservatives, emulsifiers, oils, glycols, vitamins such as vitamin E, UV filters.

8. Use according to any one of the preceding claims, **characterised in that** the composition presents itself in the form of a gel, a lotion, a cream, an emulsion, a milk, a spray.

9. Use according to any one of the preceding claims, **characterised in that** said extract is associated with an extract of sanicula europaea.

10. Use according to claim 9, **characterised in that** said extract of sanicula europaea is a water-soluble extract of the aerial parts of the plant.

11. Use according to any one of the preceding claims, **characterised in that** said extract or the association of extracts further comprises an extract of *Furcellaria lumbricalis.*

12. Use according to claim 11, **characterised in that** said extract of *Furcellaria lumbricalis* comprises oligofurcellaran enriched with sea salts.

13. Use according to any one of the preceding claims, **characterised in that** said extract or the association of extracts further comprises an extract of *Lampsana communis.*

14. Use according to claim 13, **characterised in that** said extract of *Lampsana communis* is obtained by hydro-glycolic extraction of the aerial part of the plant.

15. Method for cosmetic care, to fight against and prevent the effects of pollution on the skin, **characterised in that** an adequate quantity of an extract of *Diospyros mespiliformis* is applied by itself on the skin or in association with an extract of sanicula europaea and/or an extract of *Furcellaria lumbricalis* and/or an extract of *Lampsana communis* or of a composition comprising said extract or one of said associations.
